# EUROPEAN PATENT SPECIFICATION

(11) **EP 4 145 120 B1**
(45) Date of publication and mention of the grant of the patent: **25.12.2024**
(21) Application number: 22193545.5
(22) Date of filing: 01.09.2022
(51) Int. Cl.: G01N 27/404, G01N 27/416, G01N 33/00, G01L 1/16, G01L 1/22

(54) **MASS AND FORCE INDICATORS IN DIAGNOSTICS AND OUTPUT CORRECTION FOR ELECTROCHEMICAL GAS SENSORS**
MASSE- UND KRAFTINDIKATOREN IN DER DIAGNOSE UND AUSGANGSKORREKTUR FÜR ELEKTROCHEMISCHE GASSENSOREN
INDICATEURS DE MASSE ET DE FORCE DANS LE DIAGNOSTIC ET LA CORRECTION DE SORTIE DE CAPTEURS DE GAZ ÉLECTROCHIMIQUES

(30) Priority: 01.09.2021 US 202163239595 P
(43) Date of publication of application: 08.03.2023
(73) Proprietor: Carrier Corporation, Palm Beach Gardens, FL 33418 (US)
(72) Inventor: EVJU, Jon K., Minneapolis 55438 (US); FILLMORE, Robert L., Minneapolis 55438 (US)
(74) Representative: Dehns

(56) References cited:
- US-A- 5 580 675
- US-B2- 6 629 444

## Description

The present invention relates to gas detectors and, more particularly, to mass and force indicators in diagnostics and output correction for electrochemical gas sensors in gas detectors.

The precision and life-span of life-saving toxic gas electrochemical sensors are negatively impacted by long-term extreme environmental conditions. Loss and gain of solvent (often water) in the electrolyte solution (often sulfuric acid) can give rise to corresponding changes in sensitivity toward the target gas. For example, some electrochemical sensors will absorb water vapor in humid environments until they begin leaking/spilling sulfuric acid when the volume of electrolyte exceeds the available internal volume. In the other extreme (dry) case, the sulfuric acid electrolyte may become so concentrated that the acid attacks the materials and seals in the sensor housing, causing leaks and potentially catastrophic detector damage through corrosion. In the other extreme, the loss of electrolyte solution volume may expose the electrodes to air leading to sensor failure.

To assess the state of the sensor, voltage pulse, current pulse, and impedance spectroscopy are frequently employed; however, these techniques are indirect measurements that may fail to alert to all sensor failures (such as the extreme dry and/or leaking cases scenarios described above).

US 6 629 444 B2 discloses a method and apparatus for diagnosing defects in electrochemical gas sensors, in which the water vapor pressure of the air surrounding a sensor is suddenly changed by changing to more dry or more humid air, thereby causing a sharp change in the acidity at the working electrode, and hence, a transient current from the sensor. The response of the sensor to the change in water vapor pressure is monitored and is used for sensor diagnostics.

According to a first aspect of the invention, there is provided a method of using mass and force indicators in electrochemical gas sensor management. The method includes measuring a change in mass of an electrochemical gas sensor and determining a status of the electrochemical gas sensor based on results of the measuring of the change in mass.

The measuring of the change in mass may include attaching a mass sensor to the electrochemical gas sensor and mounting the electrochemical gas sensor with the mass sensor in a manner that engages the mass sensor.

The mass sensor may include one or more of a strain gauge assembly, a piezoelectric element, an elastic element and a flexible circuit with a built-in or integral strain gauge.

Determining the status of the electrochemical gas sensor based on the results of the measuring of the change in mass may include calibrating the electrochemical gas sensor prior to a usage thereof to determine an electrolyte sensitivity factor thereof, executing field sensitivity corrections using the results of the measuring of the change in mass to update the electrolyte sensitivity factor and calculating a field gas concentration based on the electrolyte sensitivity factor.

Calibrating the electrochemical gas sensor prior to a usage thereof may include factory calibration.

Calibrating the electrochemical gas sensor prior to a usage thereof may include calculating a response sensitivity factor of the electrochemical gas sensor, calculating an amount of an acid electrolyte in the electrochemical gas sensor and determining the electrolyte sensitivity factor from the response sensitivity factor and the amount of the acid electrolyte.

Executing of the field sensitivity corrections may include periodically executing the field sensitivity corrections.

Executing of the field sensitivity corrections may include determining whether the change in mass is within threshold limits and issuing a warning of sensor failure in an event the change in mass is not within the threshold limits.

The method may include issuing a warning of sensor failure based on a result of the calculating of the field gas concentration.

According to a second aspect of the invention, there is provided an electrochemical gas sensor assembly according to claim 8. The electrochemical gas sensor assembly includes an electrochemical gas sensor, a mass sensor coupled to the electrochemical gas sensor and configured to measure a change in a mass of the electrochemical gas sensor over time and a processing system configured to determine a status of the electrochemical gas sensor based on the change in the mass.

The electrochemical gas sensor may include an enclosure including a body defining an interior and an opening by which the interior is communicative with an exterior environment, a membrane disposed on an interior side of the opening and a filter disposed on an exterior side of the opening, a printed circuit board (PCB) affixed to an exterior of the body, electrodes operably disposed in the interior and coupled with the PCB and an electrolyte filling at least a portion of the interior around the electrodes.

The electrolyte may include an aqueous or non-aqueous fluid.

The aqueous or non-aqueous fluid may be replaceable by water in a humid environment via at least one of the filter, the opening, and the membrane; and the aqueous or non-aqueous fluid evaporates out of the interior in a dry environment via at least one of: the membrane, the opening, and the filter.

The mass sensor may be attached to the PCB.

The electrochemical gas sensor may be suspended on the mass sensor.

The mass sensor may include a deflectable member and a strain gauge.

The mass sensor may include a piezoelectric element.

The mass sensor may include an elastic element and a grid for optical reading.

The processing system may issue a warning in at least one of: in an event that the change in the mass exceeds threshold limits; and a mass corrected sensitivity of the electrochemical gas sensor decreases beyond a predefined threshold based on the change in the mass.

The electrochemical gas sensor may include a flexible circuit, wherein: the electrochemical gas sensor is suspended on the flexible circuit and the flexible circuit extends into the interior; the electrodes are coupled with contact pads of the printed circuit board (PCB); and the mass sensor is built-in or integral with the flexible circuit.

Additional features and advantages are realized through the techniques of the present disclosure. Other embodiments and aspects of the present invention are described in detail herein and are considered to fall within the scope of the claimed invention. For a better understanding of the invention with the advantages and the features, refer to the description and to the drawings.

Certain embodiments of the invention will now be described, by way of example only, and with reference to the drawings (wherein like reference numerals represent like parts), in which:
FIG. 1 is a flow diagram illustrating a method of using mass and force indicators in electrochemical gas sensor management;
FIG. 2 is a graphical flow diagram illustrating details of the method of FIG. 1;
FIG. 3 is a schematic side view of an exemplary electrochemical gas sensor assembly;
FIG. 4 is a graphical depiction of a relationship between a mass of an electrochemical gas sensor and a mass corrected sensitivity of the electrochemical gas sensor;
FIG. 5 is a schematic side view of an electrochemical gas sensor assembly;
FIG. 6 is a schematic side view of an electrochemical gas sensor assembly; and
FIG. 7 is a schematic side view of an electrochemical gas sensor assembly.

In some, less extreme cases, sensor mass changes are used to correct for the changes in sensor sensitivity to target gases as the electrolyte solution gets more or less concentrated through evaporation or absorption of moisture. These mechanisms affect the viscosity, ion mobility and gas solubility of the electrolyte solution inside the electrochemical gas sensor, which affect sensor sensitivities to target gas.

As will be described below, losses or gains of water/solvent are directly evident as mass gains or losses that correlate directly with sensitivity changes. This correlation holds over a wide range for the sensors' sensitivity. With today's light-weight polymer sensor housings, the respective change in mass can represent 15-40% of the total sensor mass and is readily measured. Combining this significant mass change with modern methods suitable for measuring the force of an object due to gravity working on it, it becomes easy to implement a direct diagnostics tool. Over most of the useful range, the direct diagnostics tool can be implemented completely without perturbing the electrochemical sensor. Toward the end of the useful life, in an environment where the electrolyte is drying out, it may become advantageous to implement additional diagnostic tools or thresholds which are well known in the prior art. The output from any or all of these diagnostic methods may be employed to warn the user of nearing sensor failure to allow replacement of sensor, or sensor assembly, minimizing downtime.

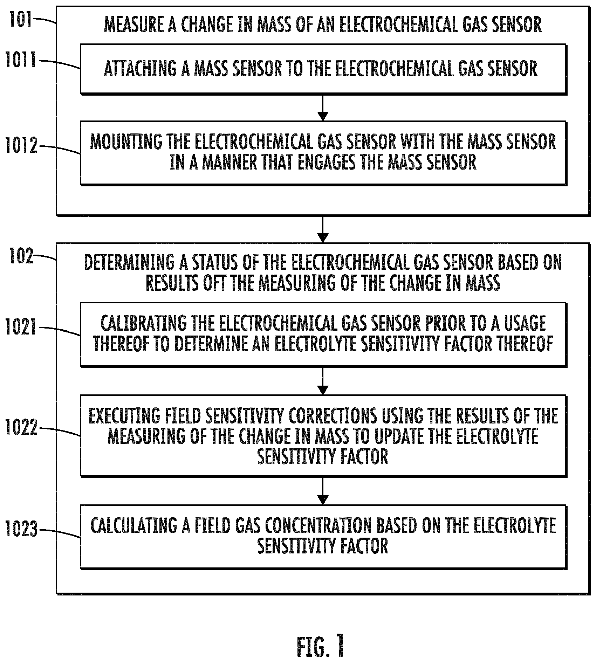

With reference to FIG. 1, a method of using mass and force indicators in electrochemical gas sensor management is provided. The method includes measuring 101 a change in mass of an electrochemical gas sensor and determining 102 a status of the electrochemical gas sensor based on results of the measuring of the change in mass. The measuring of the change in mass of operation 101 can include attaching 1011 a mass sensor to the electrochemical gas sensor, mounting 1012 the electrochemical gas sensor with the mass sensor in a manner that engages the mass sensor and measuring a mass of the unused or new electrochemical gas sensor. In accordance with embodiments, the mass sensor can include one or more of a strain gauge assembly, a piezo-electric element, and/or an elastic element. The determining of operation 102 can include calibrating 1021 (i.e., at the factory or otherwise prior to electrochemical gas sensor usage) the electrochemical gas sensor prior to a usage thereof to determine an electrolyte sensitivity factor thereof, executing 1022 field sensitivity corrections (i.e., at predefined times, intervals or periodically) using the results of the measuring of the change in mass to update the electrolyte sensitivity factor and calculating 1023 a field gas concentration based on the electrolyte sensitivity factor.

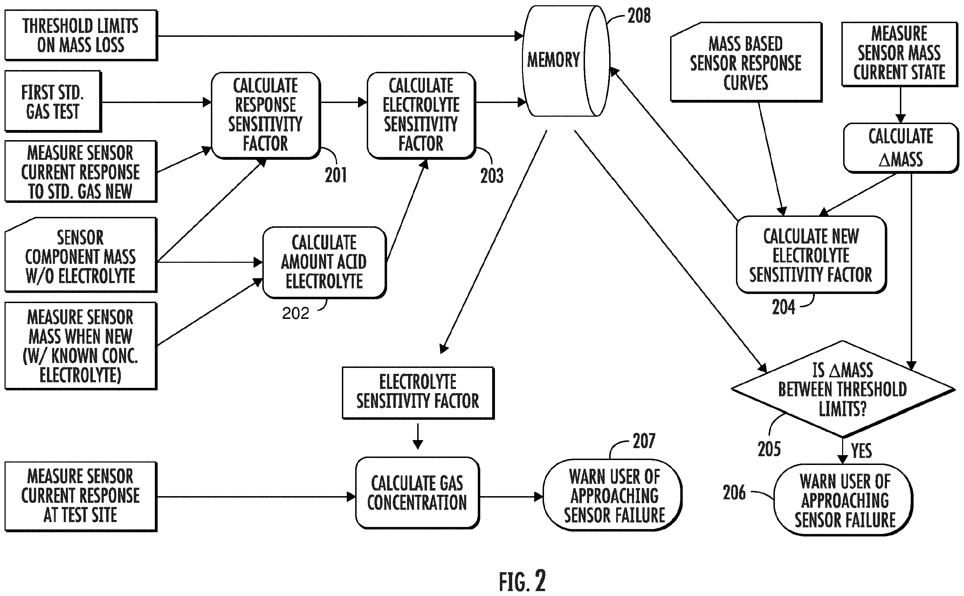

With reference to FIG. 2, further embodiments of the method of FIG. 1 are illustrated. For example, as shown, the calibrating of operation 1021 can include calculating 201 a response sensitivity factor of the electrochemical gas sensor from a sensor mass without electrolyte, from a standard gas test, and from a measurement of a sensor current response during the standard gas test. Also, the calibrating of operation 1021 can include calculating 202 an amount of an acid electrolyte in the electrochemical gas sensor from the sensor mass and the sensor mass with electrolyte at the factory or prior to usage. In addition, the calibrating of operation 1021 can include determining 203 the electrolyte sensitivity factor from the response sensitivity factor and the amount and concentration of the acid electrolyte per the measured sensor mass changes

As another example, the executing of the field sensitivity corrections using the results of the measuring of the change in mass to update the electrolyte sensitivity factor of operation 1022 can include calculating 204 a new electrolyte sensitivity factor from the change in mass and from known mass-based sensor response curves. Also, the executing of the field sensitivity corrections using the results of the measuring of the change in mass to update the electrolyte sensitivity factor of operation 1022 can include determining 205 whether the change in mass is within threshold limits or, if it is in agreement with results from standard electrochemical characterization methods, recent calibration events or historical environmental factors (Temperature, humidity and pressure) and issuing 206 a warning of sensor failure in an event the change in mass is not within the threshold limits.

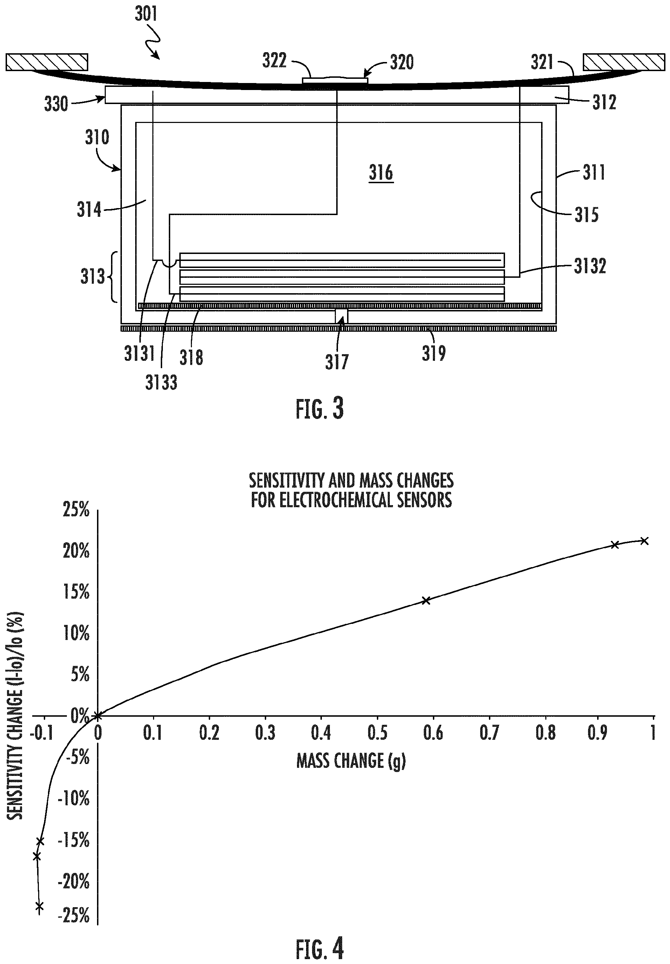

As another example, the method can further include issuing 207 a warning of sensor failure based on a result of the calculating of the field gas concentration, which takes into account the electrolyte sensitivity factor (original or updated/new) and measured sensor current response at a test site, and inputting into a memory 208 of a processing system 330 (see FIGS. 3-5) threshold limits on mass changes and original and updated/new electrolyte sensitivity factors.

With reference to FIGS. 3-6, an electrochemical gas sensor assembly 301 is provided and includes an electrochemical gas sensor 310, a mass sensor 320, and the processing system 330. The mass sensor 320 is coupled to the electrochemical gas sensor 310 and is configured to measure a change in a mass of the electrochemical gas sensor 310 over time. The processing system 330 includes the memory 208 of FIG. 2 and is configured to determine a status of the electrochemical gas sensor 310 based on the change in the mass read by the mass sensor 320.

The electrochemical gas sensor 310 includes an enclosure 311, an optional printed circuit board (PCB) 312, electrodes 313, and an electrolyte 314. The enclosure 311 includes a body 315 that is formed to define an interior 316 and one or more capillary openings (hereinafter referred to as an "opening") 317 by which the interior 316 is communicative with an exterior environment. The enclosure 311 may further include a membrane 318, which is disposed on an interior side of the opening 317, and a filter (i.e., a capillary filter) 319, which is disposed on an exterior side of the opening 317. The PCB 312 is affixed to an exterior of the body 315 and includes the memory 208 of FIG. 2 and a processor which is configured to read and to execute executable instructions stored in the memory 208 in order to execute the method(s) described herein. The electrodes 313 are operably disposed in the interior 316 and are operably coupled with the PCB 312. The electrodes 313 can include a counter electrode 3131, a reference electrode 3132 and a sensing (working) electrode 3133. The electrolyte 314 fills at least a portion of the interior 316 around the electrodes 313 and can include or be provided as an aqueous or non-aqueous fluid, such as an aqueous acid electrolyte or another similar electrolyte.

In accordance with embodiments, the electrolyte 314 can be diluted over time by water in a humid environment entering the sensor via at least one of: the filter 319, the opening 317, and the membrane 318. This increases the mass of the electrochemical gas sensor 310. Conversely, some quantity of water can evaporate out of the interior 316 in a dry environment from the electrolyte 314 via at least one of: the membrane 318, the opening 317, and the filter 319. This can decrease a mass of the electrochemical gas sensor 310.

As shown in FIG. 4, the increase or decrease in the mass of the electrochemical gas sensor 310 correlates with a sensitivity change in the electrochemical gas sensor 310. This sensitivity change needs to be taken into account when readings of the electrochemical gas sensor 310 are taken in terms of reliability. Also, the sensitivity change can be considered in terms of making a decision about issuing a warning as to the status of the electrochemical gas sensor 310 and/or as to making a decision about replacing the electrochemical gas sensor 310. As such, the ability to determine the change in the mass of the electrochemical gas sensor 310, which is provided by the mass sensor 320, is a valuable advance as compared to techniques that never considered the change in mass as being indicative of changes to gas sensor response.

The processing system 330 can be configured to issue a warning in at least one of: an event the change in the mass exceeds threshold limits, and a mass corrected sensitivity of the electrochemical gas sensor 310 decreases beyond a predefined threshold based on the change in the mass.

With continued reference to at least FIGS. 3, 5 and 6, the mass sensor 320 can be attached to the PCB 312 with the electrochemical gas sensor 310 as a whole being effectively suspended on the mass sensor 320.

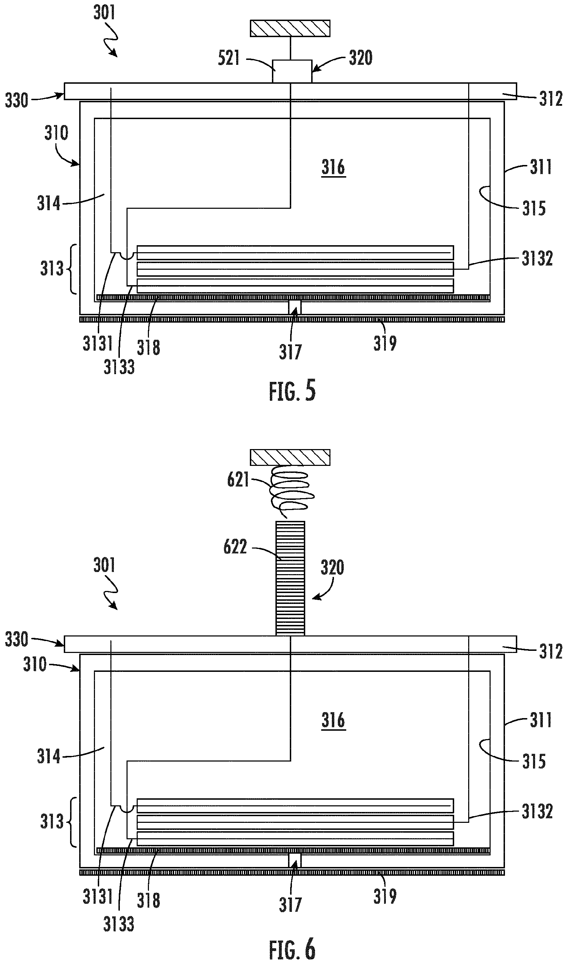

That is, as shown in FIG. 3, the mass sensor 320 can include a deflectable member 321, which can be fastened at opposite ends thereof to a roof or ceiling and affixed at a center thereof to the PCB 312, and a strain gauge 322. In this case, the deflectable member 321 deflects upwardly or downwardly based on the change in mass of the electrochemical gas sensor 310. The strain gauge 322 is capable of detecting the strain associated with this deflection and thus effectively sensing the change in the mass of the electrochemical gas sensor 310. As shown in FIG. 5, the mass sensor 320 can include a piezoelectric element 521, which can be fastened to a roof or ceiling and affixed to the PCB 312. In this case, the piezoelectric element 521 generates a current based on the change in mass of the electrochemical gas sensor 310. This current can be used to effectively sense the change in the mass of the electrochemical gas sensor 310. As shown in FIG. 6, the mass sensor 320 can include an elastic element 621 and a grid for optical reading 622 which operate similarly as the deflectable member 321 and the strain gauge 322 of FIG. 3.

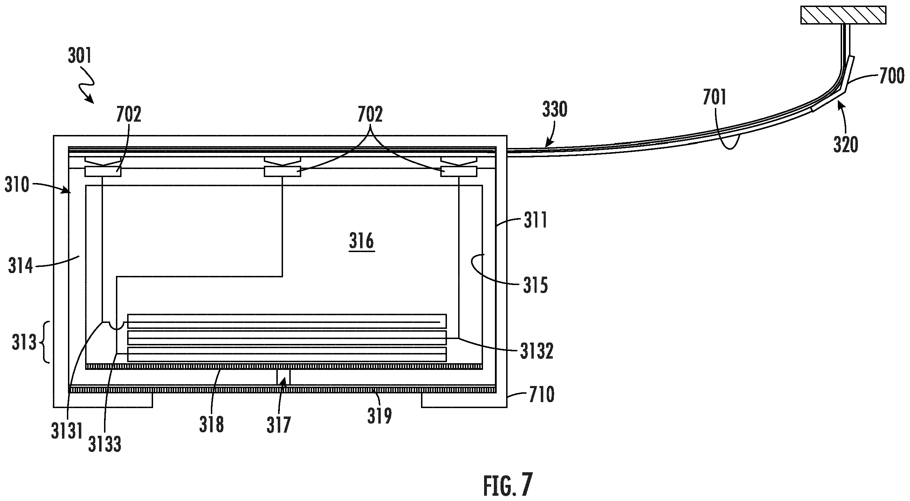

With reference to FIG. 7, the mass sensor 320 can be provided as a strain gauge 700 or another similar device and can be built-into or integral with a flexible circuit 701 that extends into the interior 316 of the body 315. In these or other cases, the electrodes 313 can be coupled with the flexible circuit 701 via contact pads 702 of the flexible circuit 701 and the processing system 330 can be incorporated into the flexible circuit 701. As shown in FIG. 7, a sensor clip or housing 710 can be provided to secure the electrochemical gas sensor 310 to the flexible circuit 701 and to ensure that there is good and reliable electrical connections between the electrodes 313 and the contact pads 702.

It should be appreciated that the mass sensor 320 may be any electrical or mechanical device capable of measuring a mass change, and, may be positioned on any suitable location capable of providing an accurate indication of a change in mass of the electrochemical gas sensor 310. It is to be understood that additional features, such as accelerometers, gyroscopes and other similar components, can be incorporated either in the sensor assembly or in the detector itself. In any case, these additional features can serve to collect data on alignment of the gas sensor (i.e., in a case of non-vertical alignment in mounting) as well as vibrations (i.e., in a case in which gas detector is mounted in an area of high, expected vibration) in order to correct gravitational measurements accordingly.

Technical effects and benefits of the present invention are the provision of systems and methods of using mass and force indicators in diagnostics and output corrections for electrochemical gas sensors in gas detectors. Since the mass and force changes translate to changes in concentration and volume of electrolyte, it is possible to assess the electrochemical sensors' sensitivity and to adjust detector output accordingly and to determine if the sensor is approaching or passing a threshold where a replacement should be made. It is also relatively easy to set thresholds to trigger sensor replacements before the sensors begin leaking or before the sensor dries out to expose an electrode to air.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description, but is not intended to be exhaustive or limited to the technical concepts in the form disclosed. Many modifications and variations will be apparent to those of ordinary skill in the art without departing from the scope of the invention as defined in the claims. The embodiments were chosen and described in order to explain the principles of the invention and the practical application thereof, and to enable others of ordinary skill in the art to understand the disclosure for various embodiments with various modifications as are suited to the particular use contemplated.

## Claims

1. A method of using mass and force indicators in electrochemical gas sensor management, the method comprising:
measuring (101) a change in mass of an electrochemical gas sensor (310); and
determining (102) a status of the electrochemical gas sensor (310) based on results of the measuring of the change in mass.

2. The method according to claim 1, wherein the measuring (101) of the change in mass comprises:
attaching (1011) a mass sensor (320) to the electrochemical gas sensor (310); and
mounting (1012) the electrochemical gas sensor (310) with the mass sensor (320) in a manner that engages the mass sensor (320).

3. The method according to claim 2, wherein the mass sensor (320) comprises one or more of a strain gauge assembly (322), a piezo-electric element (521), an elastic element (621), and a flexible circuit (701) with a built-in or integral strain gauge (700).

4. The method according to any preceding claim, wherein the determining (102) comprises:
calibrating (1021) the electrochemical gas sensor (310) prior to a usage thereof to determine an electrolyte sensitivity factor thereof;
executing (1022) field sensitivity corrections using the results of the measuring of the change in mass to update the electrolyte sensitivity factor; and
calculating (1023) a field gas concentration based on the electrolyte sensitivity factor.

5. The method according to claim 4, wherein the calibrating (1021) comprises factory calibration; and/or wherein the calibrating (1021) comprises:
calculating (201) a response sensitivity factor of the electrochemical gas sensor (310);
calculating (202) an amount of an acid electrolyte in the electrochemical gas sensor (310); and
determining (203) the electrolyte sensitivity factor from the response sensitivity factor and the amount of the acid electrolyte.

6. The method according to claim 4 or 5, wherein the executing (1022) of the field sensitivity corrections comprises periodically executing the field sensitivity corrections and/or wherein the executing (1022) of the field sensitivity corrections comprises:
determining (205) whether the change in mass is within threshold limits; and
issuing (206) a warning of sensor failure in an event the change in mass is not within the threshold limits.

7. The method according to claim 4, 5 or 6, further comprising issuing (207) a warning of sensor failure based on a result of the calculating of the field gas concentration.

8. An electrochemical gas sensor assembly (301), comprising:
an electrochemical gas sensor (310); and
a processing system (330) configured to determine a status of the electrochemical gas sensor (310);
**characterised by**:
a mass sensor (320) coupled to the electrochemical gas sensor (310) and configured to measure a change in a mass of the electrochemical gas sensor (310) over time;
wherein the processing system (330) is configured to determine the status of the electrochemical gas sensor (310) based on the change in the mass.

9. The electrochemical gas sensor assembly (301) according to claim 8, wherein the electrochemical gas sensor (310) comprises:
an enclosure (311) comprising a body (315) defining an interior (316) and an opening (317) by which the interior (316) is communicative with an exterior environment, a membrane (318) disposed on an interior side (316) of the opening (317) and a filter (319) disposed on an exterior side of the opening (317);
a printed circuit board (PCB) (312) affixed to an exterior of the body (315);
electrodes (313) operably disposed in the interior (316) and coupled with the PCB (312); and
an electrolyte (314) filling at least a portion of the interior (316) around the electrodes (313).

10. The electrochemical gas sensor assembly (301) according to claim 9, wherein the electrolyte (314) comprises an aqueous or non-aqueous fluid; optionally wherein:
the aqueous or non-aqueous fluid is replaceable by water in a humid environment via at least one of: the filter (319), the opening (317), and the membrane (318), and
the aqueous or non-aqueous fluid evaporates out of the interior (316) in a dry environment via at least one of: the membrane (318), the opening (317), and the filter (319).

11. The electrochemical gas sensor assembly (301) according to claim 9 or 10, wherein the mass sensor (320) is attached to the PCB (312); and/or wherein the electrochemical gas sensor (310) is suspended on the sensor (320).

12. The electrochemical gas sensor assembly (301) according to claim 8, 9, 10 or 11, wherein the mass sensor (320) comprises a deflectable member (321) and a strain gauge (322); and/or
wherein the mass sensor (320) comprises a piezoelectric element (521); and/or
wherein the mass sensor (320) comprises an elastic element (621) and a grid for optical reading (622).

13. The electrochemical gas sensor assembly (301) according to claim 8, 9, 10, 11 or 12, wherein the processing system (330) issues a warning in at least one of:
an event the change in the mass exceeds threshold limits, and
a mass corrected sensitivity of the electrochemical gas sensor (310) decreases beyond a predefined threshold based on the change in the mass.

14. The electrochemical gas sensor assembly (301) according to claim 9 or 10, comprising:
a flexible circuit (701); wherein:
the electrochemical gas sensor (310) is suspended on the flexible circuit (701) and the flexible circuit (701) extends into the interior (316);
the electrodes (313) are coupled with contact pads of the PCB (312); and
the mass sensor (320) is built-in or integral with the flexible circuit (701).

## Patentansprüche

1. Verfahren zur Verwendung von Massen- und Kraftindikatoren bei der Verwaltung eines elektrochemischen Gassensors, wobei das Verfahren umfasst:
Messen (101) einer Massenänderung eines elektrochemischen Gassensors (310); und Bestimmen (102) eines Status des elektrochemischen Gassensors (310) basierend auf Ergebnissen der Messung der Massenänderung.

2. Verfahren nach Anspruch 1, wobei das Messen (101) der Massenänderung umfasst:
Befestigen (1011) eines Massensensors (320) an dem elektrochemischen Gassensor (310); und
Montieren (1012) des elektrochemischen Gassensors (310) mit dem Massensensor (320) in einer Weise, die den Massensensor (320) in Eingriff bringt.

3. Verfahren nach Anspruch 2, wobei der Massensensor (320) eines oder mehrere von einer Dehnungsmessstreifenanordnung (322), einem piezoelektrischen Element (521), einem elastischen Element (621) und einer flexiblen Schaltung (701) mit einem eingebauten oder integrierten Dehnungsmessstreifen (700) umfasst.

4. Verfahren nach einem vorstehenden Anspruch, wobei das Bestimmen (102) umfasst:
Kalibrieren (1021) des elektrochemischen Gassensors (310) vor seiner Verwendung, um seinen Elektrolytempfindlichkeitsfaktor zu bestimmen;
Ausführen (1022) von Feldempfindlichkeitskorrekturen unter Verwendung der Ergebnisse der Messung der Massenänderung, um den Elektrolytempfindlichkeitsfaktor zu aktualisieren; und
Berechnen (1023) einer Feldgaskonzentration basierend auf dem Elektrolytempfindlichkeitsfaktor.

5. Verfahren nach Anspruch 4, wobei das Kalibrieren (1021) eine Werkskalibrierung umfasst; und/oder wobei das Kalibrieren (1021) umfasst:
Berechnen (201) eines Ansprechempfindlichkeitsfaktors des elektrochemischen Gassensors (310);
Berechnen (202) einer Menge eines sauren Elektrolyten in dem elektrochemischen Gassensor (310); und
Bestimmen (203) des Elektrolytempfindlichkeitsfaktors aus dem Ansprechempfindlichkeitsfaktor und der Menge des sauren Elektrolyten.

6. Verfahren nach Anspruch 4 oder 5, wobei das Ausführen (1022) der Feldempfindlichkeitskorrekturen das periodische Ausführen der Feldempfindlichkeitskorrekturen umfasst und/oder wobei das Ausführen (1022) der Feldempfindlichkeitskorrekturen umfasst:
Bestimmen (205), ob die Massenänderung innerhalb von Schwellengrenzen liegt; und
Ausgeben (206) einer Sensorausfallwarnung für den Fall, dass die Massenänderung nicht innerhalb der Schwellengrenzen liegt.

7. Verfahren nach Anspruch 4, 5 oder 6, das weiter das Ausgeben (207) einer Sensorausfallwarnung basierend auf einem Ergebnis des Berechnens der Feldgaskonzentration umfasst.

8. Elektrochemische Gassensoranordnung (301), umfassend:
einen elektrochemischen Gassensor (310); und
ein Verarbeitungssystem (330), das konfiguriert ist, um einen Status des elektrochemischen Gassensors (310) zu bestimmen;
**gekennzeichnet durch**:
einen Massensensor (320), der mit dem elektrochemischen Gassensor (310) gekoppelt und konfiguriert ist, um eine Massenänderung des elektrochemischen Gassensors (310) über die Zeit hinweg zu messen;
wobei das Verarbeitungssystem (330) konfiguriert ist, um den Status des elektrochemischen Gassensors (310) basierend auf der Massenänderung zu bestimmen.

9. Elektrochemische Gassensoranordnung (301) nach Anspruch 8, wobei der elektrochemische Gassensor (310) umfasst:
einen Einschluss (311), der einen Körper (315) umfasst, der einen Innenraum (316) und eine Öffnung (317) definiert, durch die der Innenraum (316) mit einer Außenumgebung in Verbindung steht, eine Membran (318), die an einer Innenseite (316) der Öffnung (317) angeordnet ist, und einen Filter (319), der an einer Außenseite der Öffnung (317) angeordnet ist;
eine gedruckte Leiterplatte (PCB) (312), die an einer Außenseite des Körpers (315) fixiert ist;
Elektroden (313), die betriebsfähig in dem Innenraum (316) angeordnet, und mit der PCB (312) gekoppelt sind; und
einen Elektrolyt (314), der mindestens einen Abschnitt des Innenraums (316) um die Elektroden (313) herum füllt.

10. Elektrochemische Gassensoranordnung (301) nach Anspruch 9, wobei der Elektrolyt (314) ein wässriges oder nicht-wässriges Fluid umfasst; wobei optional:
das wässrige oder nicht-wässrige Fluid in einer feuchten Umgebung über mindestens eines durch Wasser ersetzbar ist, von: dem Filter (319), der Öffnung (317) und der Membran (318), und
das wässrige oder nicht-wässrige Fluid aus dem Innenraum (316) in einer trockenen Umgebung über mindestens eines verdunstet von: der Membran (318), der Öffnung (317) und dem Filter (319).

11. Elektrochemische Gassensoranordnung (301) nach Anspruch 9 oder 10, wobei der Massensensor (320) an der PCB (312) befestigt ist; und/oder wobei der elektrochemische Gassensor (310) an dem Sensor (320) aufgehängt ist.

12. Elektrochemische Gassensoranordnung (301) nach Anspruch 8, 9, 10 oder 11, wobei der Massensensor (320) ein auslenkbares Element (321) und einen Dehnungsmessstreifen (322) umfasst; und/oder
wobei der Massensensor (320) ein piezoelektrisches Element (521) umfasst; und/oder
wobei der Massensensor (320) ein elastisches Element (621) und ein Gitter zum optischen Ablesen (622) umfasst.

13. Elektrochemische Gassensoranordnung (301) nach Anspruch 8, 9, 10, 11 oder 12, wobei das Verarbeitungssystem (330) eine Warnung bei mindestens einem ausgibt, von:
einem Fall, dass die Massenänderung Schwellengrenzen überschreitet, und
einer massenkorrigierten Empfindlichkeit des elektrochemischen Gassensors (310), die basierend auf der Massenänderung unter eine vordefinierte Schwelle sinkt.

14. Elektrochemische Gassensoranordnung (301) nach Anspruch 9 oder 10, umfassend:
eine flexible Schaltung (701); wobei:
der elektrochemische Gassensor (310) an der flexiblen Schaltung (701) aufgehängt ist und sich die flexible Schaltung (701) in den Innenraum (316) erstreckt;
die Elektroden (313) mit Kontaktflächen der PCB (312) gekoppelt sind; und
der Massensensor (320) in die flexible Schaltung (701) eingebaut oder darin integriert ist.

## Revendications

1. Procédé d'utilisation d'indicateurs de masse et de force dans une gestion de capteur de gaz électrochimique, le procédé comprenant :
la mesure (101) d'un changement de masse d'un capteur de gaz électrochimique (310) ; et la détermination (102) d'un état du capteur de gaz électrochimique (310) sur la base de résultats de la mesure du changement de masse.

2. Procédé selon la revendication 1, dans lequel la mesure (101) du changement de masse comprend :
la fixation (1011) d'un capteur de masse (320) au capteur de gaz électrochimique (310) ; et
le montage (1012) du capteur de gaz électrochimique (310) avec le capteur de masse (320) d'une manière qui met en prise le capteur de masse (320).

3. Procédé selon la revendication 2, dans lequel le capteur de masse (320) comprend un ou plusieurs d'un ensemble à jauge de contrainte (322), d'un élément piézoélectrique (521), d'un élément élastique (621) et d'un circuit flexible (701) avec une jauge de contrainte intégrée ou d'un seul tenant (700).

4. Procédé selon une quelconque revendication précédente, dans lequel la détermination (102) comprend :
l'étalonnage (1021) du capteur de gaz électrochimique (310) avant une utilisation de celui-ci pour déterminer un facteur de sensibilité électrolytique de celui-ci ;
l'exécution (1022) de corrections de sensibilité de champ à l'aide des résultats de la mesure du changement de masse pour mettre à jour le facteur de sensibilité électrolytique ; et
le calcul (1023) d'une concentration de gaz de champ sur la base du facteur de sensibilité électrolytique.

5. Procédé selon la revendication 4, dans lequel l'étalonnage (1021) comprend un étalonnage en usine ; et/ou dans lequel l'étalonnage (1021) comprend :
le calcul (201) d'un facteur de sensibilité de réponse du capteur de gaz électrochimique (310) ;
le calcul (202) d'une quantité d'un électrolyte acide dans le capteur de gaz électrochimique (310) ; et
la détermination (203) du facteur de sensibilité électrolytique à partir du facteur de sensibilité de réponse et de la quantité de l'électrolyte acide.

6. Procédé selon la revendication 4 ou 5, dans lequel l'exécution (1022) des corrections de sensibilité de champ comprend l'exécution périodique des corrections de sensibilité de champ et/ou dans lequel l'exécution (1022) des corrections de sensibilité de champ comprend :
la détermination (205) pour savoir si le changement de masse se situe dans des limites du seuil ; et
l'émission (206) d'un avertissement de défaillance de capteur au cas où un changement de masse ne se situe pas dans les limites de seuil.

7. Procédé selon la revendication 4, 5 ou 6, comprenant en outre l'émission (207) d'un avertissement de défaillance de capteur sur la base d'un résultat du calcul de la concentration de gaz de champ.

8. Ensemble capteur de gaz électrochimique (301), comprenant :
un capteur de gaz électrochimique (310) ; et
un système de traitement (330) configuré pour déterminer un état du capteur de gaz électrochimique (310) ;
**caractérisé par** :
un capteur de masse (320) couplé au capteur de gaz électrochimique (310) et configuré pour mesurer un changement de masse du capteur de gaz électrochimique (310) au fil du temps ;
dans lequel le système de traitement (330) est configuré pour déterminer l'état du capteur de gaz électrochimique (310) en fonction du changement de masse.

9. Ensemble capteur de gaz électrochimique (301) selon la revendication 8, dans lequel le capteur de gaz électrochimique (310) comprend :
une enceinte (311) comprenant un corps (315) définissant un intérieur (316) et une ouverture (317) par laquelle l'intérieur (316) communique avec un environnement extérieur, une membrane (318) disposée sur un côté intérieur (316) de l'ouverture (317) et un filtre (319) disposé sur un côté extérieur de l'ouverture (317) ;
une carte de circuit imprimé (PCB) (312) fixée sur un extérieur du corps (315) ;
des électrodes (313) disposées de manière fonctionnelle dans l'intérieur (316) de la PCB (312) et couplées à celle-ci ; et
un électrolyte (314) remplissant au moins une partie de l'intérieur (316) autour des électrodes (313).

10. Ensemble capteur de gaz électrochimique (301) selon la revendication 9, dans lequel l'électrolyte (314) comprend un fluide aqueux ou non aqueux ; facultativement dans lequel :
le fluide aqueux ou non aqueux peut être remplacé par de l'eau dans un environnement humide par le biais d'au moins un : du filtre (319), de l'ouverture (317) et de la membrane (318), et
le fluide aqueux ou non aqueux s'évapore hors de l'intérieur (316) dans un environnement sec par le biais d'au moins un : de la membrane (318), de l'ouverture (317) et du filtre (319).

11. Ensemble capteur de gaz électrochimique (301) selon la revendication 9 ou 10, dans lequel le capteur de masse (320) est fixé à la PCB (312) ; et/ou dans lequel le capteur de gaz électrochimique (310) est suspendu au capteur (320).

12. Ensemble capteur de gaz électrochimique (301) selon la revendication 8, 9, 10 ou 11, dans lequel le capteur de masse (320) comprend un élément déformable (321) et une jauge de contrainte (322) ; et/ou
dans lequel le capteur de masse (320) comprend un élément piézoélectrique (521) ; et/ou
dans lequel le capteur de masse (320) comprend un élément élastique (621) et une grille de lecture optique (622).

13. Ensemble capteur de gaz électrochimique (301) selon la revendication 8, 9, 10, 11 ou 12, dans lequel le système de traitement (330) émet un avertissement dans au moins un :
d'un cas où le changement de masse dépasse des limites de seuil, et
d'une sensibilité de masse corrigée du capteur de gaz électrochimique (310) qui diminue au-delà d'un seuil prédéfini en fonction du changement de masse.

14. Ensemble capteur de gaz électrochimique (301) selon la revendication 9 ou 10, comprenant :
un circuit flexible (701) ; dans lequel :
le capteur de gaz électrochimique (310) est suspendu sur le circuit flexible (701) et le circuit flexible (701) s'étend à l'intérieur (316) ;
les électrodes (313) sont couplées à des plots de contact de la PCB (312) ; et
le capteur de masse (320) est intégré ou d'un seul tenant avec le circuit flexible (701).
